# EUROPEAN PATENT SPECIFICATION

(11) **EP 3 350 312 B1**
(45) Date of publication and mention of the grant of the patent: **16.12.2020**
(21) Application number: 15779014.8
(22) Date of filing: 15.09.2015
(51) Int. Cl.: C12N 5/071, A61K 35/36, A61L 27/60, A61P 17/02, C12M 3/00

(54) **A METHOD OF CULTURING CELLS**
VERFAHREN ZUR KULTIVIERUNG VON ZELLEN
PROCÉDÉ DE CULTURE DE CELLULES

(43) Date of publication of application: 25.07.2018
(73) Proprietor: Stellenbosch University, 7600 Western Cape Province (ZA)
(72) Inventor: KLEINTJES, Wayne George, 7500 Cape Town (ZA)
(74) Representative: Williams Powell
(86) International application number: PCT/IB2015/057070
(87) International publication number: WO 2017/046629

(56) References cited:
- WO-A2-2008/060822
- US-A- 4 617 326
- US-A- 6 039 972
- US-A1- 2007 122 906
- US-A1- 2013 072 565
- Butcher M: "DACC antimicrobial technology: a new paradigm in bioburden management", Journal of Wound Care, May 2011 (2011-05), pages 1-19, XP055275991, Retrieved from the Internet: URL:http://cutimed.com/fileadmin/templates /PDF/DACCsupp_paradigm.pdf [retrieved on 2016-05-27]
- HAN TONG ET AL: "Combining platelet-rich plasma and tissue-engineered skin in the treatment of large skin wound.", THE JOURNAL OF CRANIOFACIAL SURGERY, vol. 23, no. 2, March 2012 (2012-03), pages 439-447, XP008180407, ISSN: 1536-3732

## Description

### FIELD OF THE INVENTION

This invention relates to a method of culturing cells and in particular to a method for culturing autogenous cells for preparing cultured epithelial autografts (CEA), and discloses a method of treating a mammalian patient by transplanting autogenous cells cultured by the method of the present invention, and in particular for treating burn victims.

### BACKGROUND TO THE INVENTION

The first Cultured Epithelial Autograft (CEA) was performed in Rheinwald and Green (Serial cultivation of strains of human epidermal keratinocytes: the formation of keratinizing colonies from single cells, Cell. 1975, 6:331-344). Since then skin grafts from cultured cells have been frequently used in treating skin defects such as burn wounds.

There are, however, some associated drawbacks which have not been fully addressed such as for example, fragile epidermal sheets and structural damage which may be sustained during transport of the grafts from a culture environment to the patient, poor "graft take", microbiological contaminations etc.

Commercially available cultured epithelial autograft (CEA) products such as EPICEL have been put to good use in treating severely burned victims. A potential issue with commercially available products is that they make use of animal products which may not always be immunologically compatible with human patients. Products like EPICEL have overcome some of these compatibility issues, but at a huge expense making this product prohibitively expensive, especially in regions like Africa and other third world countries where patients struggle to afford basic health care treatment.

Where the commercial product is not readily available, it may be necessary to culture skin locally. The proximity of suitable laboratories to hospitals does not always make this a feasible option in the light of the practical concerns around the sterility and structural integrity during transport of such grafted cells.

Barlow (US Patent No 6,039,972) describes a conformable wound dressing which is prepared by producing a sub-confluent layer of cultured epithelial cells anchored to the surface of a synthetic polymeric film which is hydrophobic, non-inhibitory to cell growth and non-cytotoxic. The film requires apertures, which are formed by perforations before or after cell culture. The growth of cells takes place in a generally used growth medium containing foetal calf serum.

Rennekampf et al. (Wound closure with human keratinocytes cultured on a polyurethane dressing overlaid on a cultured human dermal replacement, Surgery, 1996, 120:16-22) described the culturing of human keratinocyte (HK) sheets to a single layer on a polyurethane sheet, which is a synthetic hydrophilic dressing. The HK was obtained from human cadaveric donor skin and achieved a positive graft take in only 61% of the animals tested.

Van Dorp et al. (A modified culture system for epidermal cells for grafting purposes: an in vitro and in vivo study, Wound Repair Regen. 1999, 7(4):214-225) cultured human and porcine epidermal keratinocytes on polyester filler substrates. However, they found successful epithelium regeneration was only possible when the cell sheets were then detached from the substrate by enzyme treatment using Dispase. Enzymatic detachment using Dispase has been known to destroy certain cells which may be useful in the wound healing process.

Hernon et al. (Clinical experience using cultured epithelial autografts leads to an alternative methodology for transferring skin cells from the laboratory to the patient, Regenerative Med. 2006, 1(6):809-821) describe the use of a chemically defined surface for the culture of keratinocytes which could subsequently be transferred to the wound bed, without the need to enzymatically detach the cells from the surface. However, this method requires a fairly complex additional step of plasma polymerisation in which material containing acid groups are deposited onto a commercial medical grade polymer. The material is introduced in a gaseous phase and a plasma energy field is created by applying a voltage over a low vacuum field.

Atiyeh and Costagliola (Cultured Epithelial autograft (CEA) in burn treatment: Three decades later, Burns, 2007, 33:405-413) describe the use of new delivery systems to transfer keratinocytes, by growing the cells on a transplantable membrane, such as synthetic polymers, that can be transferred directly to the wound bed. The effects of these polymers on the quality of the cultured cells have, however, not been fully evaluated.

Chua et al. (In vitro evaluation of fibrin mat and Tegaderm™ wound dressing for the delivery of keratinocytes- Implications of their use to treat burns, Burns, 2008, 34:175-180) describes the use of Tegaderm™, a polyurethane based wound dressing, as a substrate for cell growth. The cells were grown on a feeder layer of irradiated 3T3-J2 cells in growth medium containing 10% fetal bovine serum and it was found that the cells grown on the Tegaderm™ show a weaker expression of some cells compared to cells grown on a fibrin mat, which provides a generally better environment for the cells.

De Corte et al. (Feeder layer- and animal product-free culture of neonatal foreskin keratinocytes: improved performance, usability, quality and safety, Cell Tissue Bank, 2012, 13:175-189) mention a silicone membrane delivery technology as a way of delivering subconfluent cells whilst also providing a wound cover.

Dahlquist (US Patent No. 8,658,851) and Iwamoto et al. (EP1 637 145) describe cells grown on flexible support comprising a hydrophobic polymer and uses cells derived from a subject that needs to be treated to avoid adverse immunological reactions.

Falk and Ivarsson (Effect of a DACC dressing on the growth properties and proliferation rate of cultured fibroblasts, Journal of Wound Care, 2012, 21(7):327-332) studied the morphology and proliferation of human dermal fibroblasts using an experimental *in vitro* wound model. However, they used only commercially available fibroblasts and no other skin cell components like epidermal and dermal cells which would be required in a viable skin graft. They also made use of a growth medium containing 10% foetal calf serum.

Smith (US 2013/0072565) also makes use of hydrophobic substances such as dialkyl carbamoyl chloride (DACC) or alkyl ketene dimer (preferably associated with a carrier) to increase cell proliferation for growing commercially available human dermal fibroblasts using commercially available growth medium supplemented with 10% calf serum.

De Moraes et al. (Use of autologous fibrin glue in dermatologic surgery: application of skin graft and second intention healing, Rev Paul Med, 1998, 116(4):1747-1752) describe the production of fibrin glue (as a tissue adhesive in skin grafts) using a sample of autogenous blood.

Mishra (US Patent Application No. 2007/0122906) describes the use of a blood component such as platelet rich plasma (PRP) in a cell culture medium to grow and proliferate cells. However, the only support for the cells described, which serves to hold the cells together and direct development of mature tissue, is collagen which is generally extracted from tissue of young animals and may therefore result in immunological compatibility issues with a patient.

Scuderi et al. (Clinical application of autologous three-cellular cultured skin substitutes (CSS), in vivo, 2009, 23:991-1004) describe the use of a hyaluronic acid biomaterial scaffold on which human fibroblasts are grown, and in which the cultures media is enriched with, amongst other things, 10% of the patient's own blood serum. As the scaffold is derived from a biological source, there is also a rick of compatibility issues.

### SUMMARY OF THE INVENTION

According to an aspect of the present invention there is provided a method of culturing autogenous cells for use in the treatment of a mammalian patient as specified in claim 1.

Further features provide for substrate material to be used as a transfer dressing for transferring the cultured cells to the patient; and for the substrate material to be used as a wound cover dressing; for the substrate material to comprise a cellulose acetate, cotton gauze or nonwoven fabric; for the fatty acid to be dialkyl carbamoyl chloride (DACC), such as dihexadecyl-carbamoyl chloride or dioctadecyl-carbamoyl chloride, and/or alkyl ketene dimer (AKD); for the substrate material to be one of the CUTIMED SORBACT® range of products.

Further features provide for the cells to be skin cells or epithelial cells, for the cells to include fibroblasts and melanocytes.

Yet further features provide for the autogenous plasma to be obtained from a centrifuged blood sample of the patient and to be platelet rich plasma (PRP); and for the plasma to be applied regularly, preferably daily.

Still further features provide for the cells to be moisturised using a hydrogel, preferably every 2 to 4 days, and most preferably every 3 days.

The method may be further characterised in that no non-autogenous animal products are used in the process of growing the autogenous cultured cells and the cells cultured on the hydrophobic surface are relatively insensitive to non-sterile conditions.

Also disclosed is a method of treating a mammalian patient by transplanting autogenous cells, cultured *in vitro,* including the steps of:
a. Obtaining cells from the patient in a biopsy;
b. Providing a substrate material having a surface treated with a fatty acid ester so as to have a strong hydrophobic surface;
c. Placing the cells on the substrate material and culturing autogenous cells thereon, using autogenous plasma obtained from a blood sample of the patient as a growth medium in order to culture the cells ;
d. Transplanting the autogenous cultured cells onto the patient, using the substrate material as a transfer dressing and wound cover dressing.

The autogenous cells may be skin cells, preferably epithelial cells, for the cells to include fibroblasts and melanocytes.

The autogenous plasma may be obtained from a centrifuged blood sample of the patient and may be platelet rich plasma (PRP); for the plasma to be applied regularly, preferably daily.

The cells may be moisturised using a hydrogel, preferably every 2 to 4 days, and most preferably every 3 days.

According to another aspect of the present invention there is provided a kit for culturing autogenous cells for use in the treatment of a mammalian patient as specified in claim 9.

Further features provide for the kit to contain a plurality of syringes for extracting plasma from a blood sample obtained from the patient; a plurality of sterile needles and 15/0 blades (theatre stock).

Yet further features provide for the kit to be in the form of a partitioned container with a plurality of zones; for a first zone to contain the trypsin solution or solution precursors, for a second zone to contain the substrate material and optionally a supporting frame for holding the substrate material in a flattened condition, for a third zone to contain the tubes, preferably acid-citrate-dextrose (ACD) tubes, and for a fourth zone to contain the syringes, sterile needles and blades and a tube of hydrogel; for each zone to be individually sealed by a peel away film cover.

The kit may further include a package insert containing instructions using the kit to culture autogenous cells, for example instructions for preparing the trypsin solution from the solution precursors, directions for applying the PRP to the cells placed on the substrate material and for moisturising the cells thereon using the hydrogel, and for applying the hydrophobic substrate containing a layer of cells thereon on a patient's wound.

Also disclosed is a system for culturing autogenous cells for use in the treatment of a mammalian patient, the system including:
a. an incubator having a plurality of substrate zones, each zone being associated with a patient and being configured to receive a substrate material having cells obtained from the associated patient placed thereon so as to culture autogenous cells thereon;
b. an identifying component for identifying the patient associated with the substrate zone; and,
c. an applicator unit for operatively applying, as a growth medium in order to culture cells, autogenous plasma obtained from a blood sample of the patient to cells on a substrate material contained in the substrate zone associated with the patient.

The applicator unit may include a robotic arm operable to select a dosage unit associated with the patient, the dosage unit containing the autogenous plasma obtained from a blood sample of the patient, and the applicator unit may apply the autogenous plasma contained in the selected dosage unit.

The system may include a dosage unit bank including a plurality of dosage unit zones each of which being configured to hold a dosage unit, each dosage unit zone being associated with a patient, the identifying component may be operable to identify a dosage unit zone associated with the patient and the robotic arm may be operable to select and extract a dosage unit from the identified dosage unit zone.

The system may include a plurality of dosage units, each of which including an outlet and a plunger, and the robotic arm may be operable to position the outlet proximate the substrate zone associated with the patient and to urge the plunger towards the outlet to operatively apply autogenous plasma contained therein to cells on a substrate material contained within the substrate zone. The dosage unit may be an acid-citrate-dextrose (ACD) dosage unit.

The identifying component may maintain a look-up table in which each substrate zone is associated with coordinates and with a patient, and the identifying component may include a tracking component to track the position of the applicator unit relative to the coordinates of each substrate zone, and the identifying component may identify the patient associated with a substrate zone by comparing the position of the applicator unit to the coordinates of the substrate zones stored in the look-up table so as to identify the substrate zone to which the applicator unit is proximate and to thereby to identify the patient associated with the substrate zone.

The identifying component may maintain a look-up table in which each dosage unit zone is associated with coordinates and with a patient, and the tracking component may track the position of the applicator unit relative to the coordinates of each dosage unit zone, and the identifying component may identify the dosage unit zone associated with the patient by comparing the position of the applicator unit to the coordinates of the dosage unit zones stored in the look-up table so as to identify the dosage unit zone to which the applicator unit is proximate and to thereby to identify the dosage unit zone associated with the patient.

The incubator may be temperature controlled, and a temperature may be selected from the range of 30 degrees Celsius to 38 degrees Celsius to be maintained, preferably 37 degrees Celsius.

Aspects of the invention will now described, by way of example only, with reference to the accompanying drawings.

### BRIEF DESCRIPTION OF THE FIGURES

In the drawings:
- Figure 1: is an illustration of one embodiment of a kit for culturing autogenous cells for use in the treatment of a mammalian patient; and
- Figure 2: is a schematic diagram which illustrates an exemplary system for culturing autogenous cells for use in the treatment of a mammalian patient.

### DETAILED DESCRIPTION OF THE INVENTION

A method of culturing autogenous cells as well as a method of treating a mammalian patient is described. The cells are cultured on a substrate material having a surface treated with a fatty acid ester so as to have a strong hydrophobic surface and autogenous plasma obtained from a blood sample of the patient is used as a growth medium in order to culture the cells. The substrate material is also used as a transfer dressing for transferring the cultured cells to the patient and as a wound cover dressing. A kit as well as a system for culturing autogenous cells using the culture method is also described.

The cells were grown on a low cost hydrophobic substrate material, in this embodiment the hydrophobic dressing known as CUTIMED SORBACT®, which served as a growth template for epithelial cells and well as a transfer medium and a wound cover dressing.

Animal products, such as bovine plasma which is commonly used as growth medium for culturing cells, were not used in the culturing process of the present invention. Instead a patient's autogenous plasma, extracted from a blood sample of the patient, was used as a growth medium to feed the cells daily. The cells were also shown to proliferate equally well in sterile and in non-sterile conditions.

Aspects of the invention will now be described in more detail with reference to the following nonlimiting examples.

### Examples

### Materials

### Equipment:

- Two incubators, a centrifuge, acid-citrate-dextrose (ACD) tubes for platelet rich plasma, a sterile instrument pack from a burns theatre, sterile gloves, 20 ml syringes, 18G and 16G needles, CUTIMED SORBACT® dressing pads and ribbon gauze, sterile green cloths, face masks, sterile theatre clothes, shoe covers and head covers, 15/0 blades (theatre stock), a light microscope, slides, fixative and loupes (3.5 x magnification 45 cm focus).

### Biological requirements:

- Autogenous (patient's own) skin biopsy: 7 x 3 cm left inguinal, autogenous blood for platelet rich plasma (PRP) (36 ml blood in 6 ml ACD tube yielded 22-25 ml of PRP) and trypsin (enzyme from beef pancreas to separate epithelial cells from dermal elements, from an anatomy laboratory).

### Method

The incubator was cleaned with a chlorhexidine solution and an alcohol solution and sterile gauze with sterile water and set to warm up to 36.8 degrees Celsius. The target temperature was 37 degrees Celsius.

A skin biopsy was transported from theatre to laboratory in a sterile specimen container, immediately after an operation to remove the skin was performed. With loupes on and using the scalpel with a number 15/0 blade, the epidermis and dermis was cut off and separated. Small fragments of epithelium were cut smaller. Some keratinocytes were scraped off without using loupes.

Boiling water was used to sterilise two specimen bottles which were used to mix Trypsin powder in sterile water. The small fragments of epithelial skin were then placed in the sterile specimen bottles with the trypsin solution to further separate the epidermal and dermal elements.

A RECELL kit was also sterilised with boiling water and used to assist in cell preparation. A small sift in the kit was used to rinse off the trypsin, and forceps were then used to pick up minute specks of epithelial cells and place them on six hydrophobic CUTIMED SORBACT® pads in the central area of the pads. Other cells were placed in the two sterilised specimen bottles.

Six tubes of blood with 6 ml in each were then centrifuged at 3500 revolutions per minutes (rpm) for 8 minutes. Thereafter the tops of the tubes were removed and the plasma was drawn up with a sterile 20 ml syringe and 18-gauge needle. Meticulous attention was given to avoiding drawing up any red blood cells which might contain pyrogenic cytokines which are pro-inflammatory, like Interleukin-1, 6 and tumour necrosis factor-alpha (TNF-alpha).

The plasma was sprayed onto the cells on the CUTIMED SORBACT® pads and into the specimen bottles containing cells. CUTIMED SORBACT® ribbon gauze was laid down between the pads to form a border for the cell growth and to avoid the cells from growing over the edges of the pads.

The cells were nourished daily with fresh PRP. Moistness in the incubator was maintained by adding sterile water to the water inlet on the outside of the incubator on a daily basis. Intrasite gel was applied with a sterile glove over the cultured epithelial autograft (CEA) every third day to prevent the cells from drying out too much.

On day 14 of the CEA growth, the target day for cell transplantation was reached, exactly 108 days after the patient's admission.

The incubator with the cultured cells was taken to theatre and the cells kept at 37 degrees Celsius. After anaesthesia commencement, 36 ml of blood for PRP was taken from the patient's A-line. The patient was washed with chlorhexidine soap and draped with sterile clothes. Wound debridement was done by gentle scraping of raw areas with a metal ruler's blunt side/tip. This was done over previously grafted xenograft areas as well as a donor site on the left medial lower leg. Haemostasis was obtained with swabs soaked in an adrenalin solution (Ringers Lactate 1 litre and 1 ampule of adrenaline and 800 mg Lignocaine). CUTIMED SORBACT® pads with the CEA were then transplanted directly onto the wounds of the back, left arm, left thigh and knee with the simultaneous application of PRP. On the patient's right arm, thigh and leg, CUTIMED SORBACT® pads were used with squirted CEA and fresh PRP. A sacral bedsore was also treated with squirted CEA from cells grown in the two sterile specimen bottles, with autogenous PRP on CUTIMED SORBACT® pads. The back dressing were fixed with IOBAN, an iodine containing film dressing. The rest of the dressings were bandaged with cling/crepe.

### Results:

A 78.16% graft take was achieved. The CEA included cells grown from deeper skin layers (not just epithelial cells as in the classic technique, but including fibroblasts and melanocytes).

### Plasma dilution studies

| **Ratio of sterile water to plasma** | **obs vol** | **gel/layer** | **fluid vol** | **gel vol** | **cell vol** | **Cell/Fluid %** | **Cell/Gel %** |
|---|---|---|---|---|---|---|---|
| 1:1 | 10mm | Fluid 10 mm | 6 ml | 0 ml | 0.1 ml | 1 | 0 |
| 0.75:1 | 10 mm + | Fluid 10 mm | 6 ml | 0 ml | 0.1 ml | 1 | 0 |
| 0.5:1 | 15mm + | Gel 9 mm | 0.4 ml | 2 ml | 0.6 ml | 6,70 | 30 |
| 0.25:1 | 10mm + | Gel 7 mm | 0.4 ml | 3 ml | 0.5 ml | 7,14 | 16,67 |
| undiluted | 15mm + | Fluid 5 mm | 4 ml | 0 ml | 0.17 ml | 3,4 | 0 |
| *uncontrolled | 46 ml | 0 | 46 ml | 0 ml | 0.6 ml | 1,3 | 0 |

| | | | | | | | |
|---|---|---|---|---|---|---|---|
| * Uncontrolled = original sample cells that were in a suspension of sterile water and Trypsin were kept in a plastic dish on a table and covered with a sterile green cloth with no temperature or humidity regulation. Plasma was added to the cells daily in small amounts. Cells grew in this 'uncontrolled' suboptimal area at room temperature (more or less 24 degrees Celsius), and under non-sterile conditions. | | | | | | | |

### Results of plasma dilution studies

1. Cells appear to be robust and easy to grow with autogenous plasma.
2. Plasma dilution can be done safely up to a ratio of 0.5:1 (sterile water: plasma) with good effect, but best results are obtained by daily feeding the cells undiluted plasma. Practically every alternative day can be skipped and cells should still survive in suspension.
3. The preferred dressing is definitely hydrophobic and not hydrophilic. Any hydrogel-addition to the back of such dressings are not good for cell growth.
4. The dual function of acting as a growth medium and anti-bacterial agent makes Sorbact the best choice.

### Cell types

| **Cell types/ fibres** | **Epidermal layers** | **Dermal layers** | **Adnexal Structures** | **Central Cell Mass** | **Membrane border** |
|---|---|---|---|---|---|
| keratinocytes, fibroblasts | Dense cell masses | Darker fibres, Elastin | No | Dense, less at edges | No |
| keratinocytes; fibroblasts, elastin | Dense cell masses | Darker fibres, Elastin | No | Dense, less at edges | Not clear |
| Fibrillar Collagen/elastin | Less dense cells | Dark and light fibrils | No | Denser at edges | Yes, 1 thick layer |
| Keratinocytes Other fibres | Outer border | Not sure, dense CM | No | Denser at edges | Yes, 1 thick layer |
| Tubular fibrils, other cells, Dermal | E cells in between F | Multiple streaky fibrils | No | Dense, less at edges | Not dark/ clear |
| keratinocytes, dermal fibres | Yes | Denser masses fibres | No | Dense, less at edges | No |
| Enc Gr. keratinocytes | tree-like controlled | Not sure | No | Dense, organized | Borders, single |
| Enc Gr. Keratinocytes, dermal cells | Yes | Yes | Possibly ducts; glands | Dense, border/epid | Yes, 4 layers |
| Enc Gr. Keratinocytes, dermal cells | Not seen | Yes | Yes | Dense, border/epid | Yes, 4 layers |
| Encapsulted cell growth areas | yes, cell masses | Not sure | No | Dense dark, shades | Not clear |
| Encapsulted areas+ Tree pattern | yes | Yes, Muscle, C & E, K | Not sure | Light coloured | Yes, 4 layers |
| Encapsulated | Yes, darker | Yes, lighter, | No | Light | Lighter grey, |
| growth. K, E & C. | areas. | transparent | | coloured | yellow |
| Encapsulated growth. K, E & C. | Yes, darker areas | Yes, lighter, transparent | Yes | Light coloured | Yes, 4 layers |
| Encapsulated growth. K, E & C. | Yes, thicker outer m | Yes, light yellow; pink | Yes. Sweat gland/hair | Light Dense organized | Yes, 4 layers |
| Encapsulated growth. K, E & C. | Yes, thicker outer m | Yes, light, translucent | Yes, clear gland, duct | Light Dense organized | Yes, 4 layers |

### Comparative Dressing Study

Dried out dressings: 3-4 months after CEA ± 3-4 days of Feeds.
1. Intrasite Conformable
   Skin can grow, but becomes interwoven/attached to the fibres from which it appears that it would be difficult to have tangential layers of skin cells forming. The fibres of the dressing are very coarse.
2. Biobrane
   Organised fibres and patterns of the dressing. No clear masses of epithelial cells seen.
3. Cutimed Sorbact Swabs
   Abundant cellular growth visible between darker fibres of the dressing.
4. Cutimed Sorbact Dressing Pads
   Upside down: Gauze at bottom: No cells seen on bottom surface. 4 x magnification. Next older cells: Top side of cells: at 4 x magnification multiple cells visible between the Sorbact fibres.
   Younger dressing 2 weeks old with CEA: at 4 x magnification abundantly much more cells visible of increased density. Cells seen growing over the Sorbact fibres and in between them.
5. Sorbact Swabs: Two weeks old.
   Even though plasma was noted to seep through the dressing pad
   Sorbact gauze cells. More abundant growth with some cells appearing brownish, others grey.
6. Hydrogel pad
   Cells don't migrate well and there is pooling of plasma.
7. Cutimed Sorbact Hydro
   Pooling of plasma not good for lateral growth of the cells.

Findings:
1. Gauze type dressings are not good for growing monolayers of cells because the cells get stuck in the fibre structure and growth is haphazard.
2. Although cells may grow on Nylon mesh like Biobrane, the growth with the method of the present invention was not detectable.
3. Abundant cellular growth was seen with the Sorbact dressings.
4. The worst performing Sorbact for cell culture was where hydrogel pads was present, which led to pooling of plasma and therefore less lateral growth, and more vertical growth stimulation.
5. The hydrophobic properties appear to be very conducive to accelerate cell growth when compared to hydrophilic dressings: The hydrogel pad did not allow plasma to seep through and caused pooling which is bad for lateral cell growth.
6. The method described above using the Sorbact dressing pad (more rigid, less prone to deformity as a result of cell contractions) is probably the best, but the Sorbact gauze is less rigid and more prone to deformity by cells.
7. One would be able to grow cells on many surfaces, but there is none known that will give dual antibacterial and growth acceleration properties with the correct amount of moisture control for effective cell culture as Sorbact.
8. Other hydrogels (such as Cutimed gel BSN) other than Intrasite gel were tested, but the contents were found to be too watery. Intrasite gel appears to have the right thickness to act as an additional moisturizer to the cells.

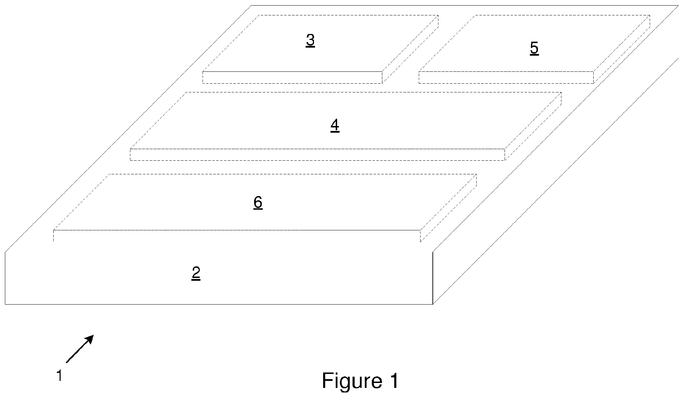

Another aspect of the invention extends to a portable kit (1) for culturing autogenous cells for use in the treatment of a mammalian patient, as illustrated in one embodiment shown in Figure 1. The kit (1) is in the form of a sterile, partitioned container (2) having a plurality of zones which include:
- a first zone (3) containing the trypsin solution or solution precursors (in the form of Trypsin powder and sterile water, which may be mixed to prepare a Trypsin solution),
- a second zone (5) containing the hydrophobic substrate material and a supporting frame for holding the substrate material in a flattened condition,
- a third zone (4) containing ACD tubes and
- a fourth zone (6) containing a plurality of syringes for extracting plasma from a blood sample obtained from the patient; a plurality of sterile needles, 15/0 blades (theatre stock), a tube of hydrogel and instructions for using the kit.

Each zone of the partitioned container (2) is individually sealed by a peel away film cover.

The Trypsin solution in the first zone (3) is used for separating epidermal and dermal cells of a biopsy obtained from a patient, as per the set of instructions contained in the fourth zone (6). The cells can be mixed with the solution directly in the first zone and the cells for culturing picked out of the zone (3) and placed onto the hydrophobic substrate material in the second zone (4).

Once a blood sample from a patient has been centrifuged and the PRP extracted, using syringes from the fourth zone (6), the plasma is placed in the ACD tubes, and can then be applied onto the cells as a growth medium on a daily basis. Application of the plasma can be performed according to set of instructions included in the fourth zone (6).

The instructions also include steps for moisturising the cells growing on the substrate material using the hydrogel, and for applying the hydrophobic substrate containing a layer of cells thereon onto a patient's wound.

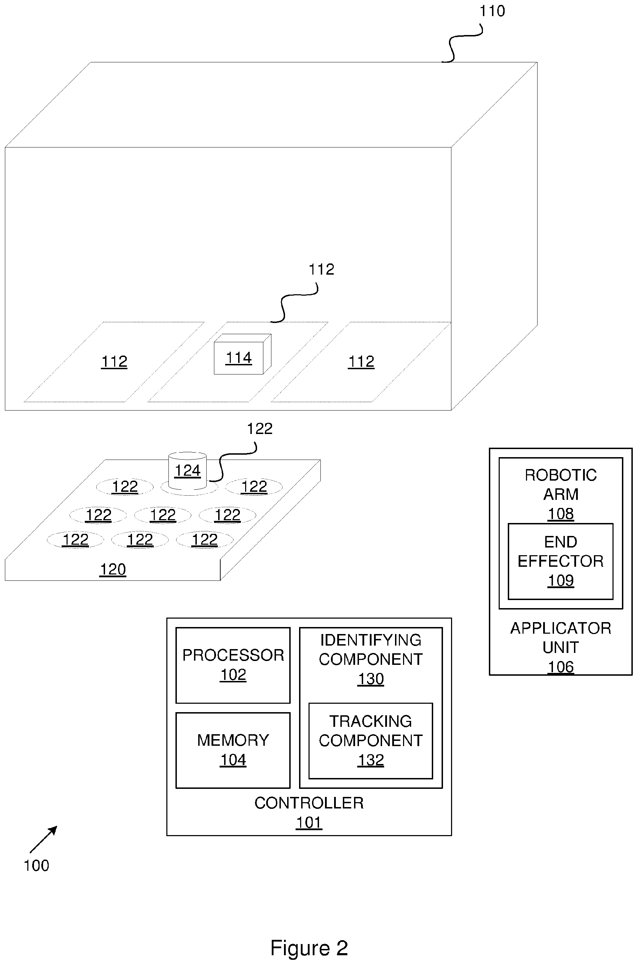

Another aspect of the disclosure is a system (100) for culturing autogenous cells for use in the treatment of a mammalian patient, one exemplary embodiment of which is illustrated in Figure 2. The system (100) includes a controller (101), having processor (102) and a memory (104), and an applicator unit (106). Code executable by the processor (102) may be stored in the memory (104) or other non-transitory computer readable medium to enable the system (100) to perform various identification, control, and applicator functions, and the like.

The system (100) includes an incubator (110) having a plurality of substrate zones (112). Each zone (112) is configured to receive a substrate material (114) having cells obtained from a patient placed thereon. Placing cells of a patient on the substrate material (114) enables autogenous cells to be cultured thereon. The incubator (110) is temperature controlled such that a temperature selected from the range of 30 degrees Celsius to 38 degrees Celsius, and preferably 37 degrees Celsius, is maintained.

The system (100) also includes a dosage unit bank (120) which includes a plurality of dosage unit zones (122). Each dosage unit zone (122) is configured to hold a dosage unit (124) which is associated with a patient. Each dosage unit (124) includes a tubular body having an outlet at a first end thereof and a plunger at a second end thereof. A chamber is defined between the plunger and the outlet and contains autogenous plasma of the patient with which the dosage unit (124) is associated. In some embodiments, the dosage unit may be an acid-citrate-dextrose (ACD) dosage unit.

The system (100) includes an identifying component (130) which maintains a look-up table in which each substrate zone (112) is logically associated with coordinates (e.g. x-, y- and z-coordinates) and with a patient (e.g. a patient identifier such a patient name or patient number). In this manner, each substrate zone (112) is associated with a patient.

The identifying component (130) is operable to identify a patient associated with the substrate zone (112) or to identify a substrate zone (112) associated with a patient. In this embodiment, the identifying component (130) includes a tracking component (132) to track the position of the applicator unit (106) relative to stored coordinates of each substrate zone (112). The identifying component (130) identifies the patient associated with a substrate zone (112) by comparing the position of the applicator unit (106) to the coordinates of the substrate zones stored in the look-up table so as to identify the substrate zone to which the applicator unit (106) is proximate and to thereby identify the patient associated with the substrate zone (112).

The identifying component (130) further maintains a look-up table in which each dosage unit zone (122) is logically associated with coordinates (e.g. x-, y- and z-coordinates) and with a patient. In this manner, each dosage unit zone (122) is associated with a patient.

The identifying component (130) is operable to identify a dosage unit zone (122) associated with the patient. The tracking component (132) is operable to track the position of the applicator unit (106) relative to the coordinates of each dosage unit zone (122). The identifying component (130) is operable to identify the dosage unit zone (122) associated with the patient by comparing the position of the applicator unit (106) to the coordinates of the dosage unit zones (122) stored in the look-up table so as to identify the dosage unit zone (122) to which the applicator unit (106) is proximate and to thereby to identify the dosage unit zone associated (122) with the patient.

The applicator unit (106) includes a robotic arm (108) having an end effector (109) which is operable to select and extract a dosage unit (124) associated with a particular patient from the dosage unit zone (122) in which it is located and to apply autogenous plasma of the patient contained therein to cells on the substrate material (114) contained in the substrate zone (112) which is associated with the patient.

In use, a particular patient may be identified. The robotic arm (108) identifies a dosage unit zone (122) which is associated with the patient. The end effector (109) removes the dosage unit (124) from the identified dosage unit zone (122). The substrate zone (112) associated with the patient is then identified and the dosage unit (124) is positioned proximate the substrate material (114) located within the substrate zone (112) and having cells obtained from the patient placed thereon. The end effector (109) urges the plunger of the dosage unit (124) towards the outlet to apply the autogenous plasma contained therein to the cells placed on a substrate material (114). The autogenous plasma, when applied to the cells on the substrate material (114) acts as a growth medium in order to culture cells.

The system (100) is configured to operate autonomously such that cells for a number of patients may be cultured on a large scale and with minimal human intervention. For example, the applicator unit may run autonomously to apply autogenous plasma of a plurality of patients to their corresponding one or more substrate materials contained within the incubator so as to culture cells on a large and ongoing scale, for example for the duration that a patient is at a facility for treatment.

It should be appreciated that the embodiment of the system described above is exemplary and that various alterations and modifications may be made thereto. For example, in another embodiment, each substrate zone and dosage unit zone may have a computer-and/or human-readable identifier or indicia (e.g. a label and/or barcode) disposed thereon. The identifier may be usable by the identifying component to identify a patient associated with each of the substrate zones and dosage unit zones.

The hydrophobic dressing used for cell grafting in the present invention, CUTIMED SORBACT® which has a hydrophobic coating made from DACC, is capable of reducing the effects of shear stress, metabolic stress and tissue infection. The DACC attracts hydrophobic bacteria and renders them inert. The Applicant postulated and found that the hydrophobic action of CUTIMED SORBACT® would allow plasma, in the form of PRP, to be applied to the substrate and the cells could grow within the plasma droplets. Dressings of this type are further described in US Patent No. 4,617,326 and US patent application no. 2006/012980.

The applicant envisages that the methods and materials of the invention are particularly beneficial in avoiding infection due to the combined anti-infective effects of the hydrophobic CUTIMED SORBACT® dressing and the use of autogenous plasma. The applicant postulates that the use of autogenous plasma allows the anti-infective effects of any antibiotic treatment, tailored specifically to the patient's bacterial profile, to persist during the *in vitro* culturing process.

Throughout the specification and claims unless the contents requires otherwise the word 'comprise' or variations such as 'comprises' or 'comprising' will be understood to imply the inclusion of a stated integer or group of integers but not the exclusion of any other integer or group of integers.

## Claims

1. A method of culturing autogenous cells for use in the treatment of a mammalian patient, the steps including:
a. Providing a substrate material having a surface treated with a fatty acid ester so as to have a strong hydrophobic surface;
b. Placing cells obtained from the patient on the hydrophobic surface of the substrate material, so as to culture autogenous cells thereon; and
c. Applying autogenous plasma obtained from a blood sample of the patient to the cells as a growth medium in order to culture the cells.

2. The method as claimed in claim 1 wherein the substrate material is used as a transfer dressing for transferring the cultured cells to the patient.

3. The method as claimed in either claim 1 or claim 2 wherein the substrate material is further used as a wound cover dressing.

4. The method as claimed in any one of the preceding claims wherein the substrate material comprises a cellulose acetate, cotton gauze or nonwoven fabric and the fatty acid is selected from a dialkyl carbamoyl chloride (DACC) and an alkyl ketene dimer (AKD).

5. The method as claimed in claim 4 wherein the DACC is dihexadecyl-carbamoyl chloride or dioctadecyl-carbamoyl chloride.

6. The method as claimed in any one of the preceding claims wherein the cells are selected from skin cells or epithelial cells which include fibroblasts and melanocytes.

7. The method as claimed in any one of the preceding claims wherein the plasma is platelet rich plasma (PRP).

8. The method as claimed in any one of the preceding claims which includes a further step of moisturising the cells using a hydrogel, at least every 2 to 4 days.

9. A kit for culturing autogenous cells for use in the treatment of a mammalian patient, the kit including:
a. a Trypsin solution or solution precursors for separating epidermal and dermal cells of a biopsy obtained from a patient
b. a substrate material having a surface treated with a fatty acid ester so as to have a strong hydrophobic surface and onto which the cells may be placed so to culture autogenous cells thereon; and
c. a plurality of tubes for containing plasma obtained from a blood sample of the patient.

10. The kit as claimed in claim 9 which further comprises a plurality of syringes for extracting plasma from a blood sample obtained from the patient; a plurality of sterile needles and 15/0 blades (theatre stock) and a tube of hydrogel.

11. The kit as claimed in claim 10 in the form of a partitioned container with a plurality of zones; including a first zone containing the trypsin solution or solution precursors; a second zone containing the substrate material and optionally a supporting frame for holding the substrate material in a flattened condition; a third zone containing the tubes; and a fourth zone containing the syringes, sterile needles and blades and hydrogel; and wherein each zone is individually sealed by a peel away film cover.

12. The kit as claimed in any one of claims 9 to 11 which further includes a package insert containing instructions for using the kit for culturing autogenous cells.

## Patentansprüche

1. Verfahren zum Kultivieren autogener Zellen zur Verwendung bei der Behandlung eines Säugetierpatienten, aufweisend die Schritte:
a. Bereitstellen eines Substratmaterials mit einer Oberfläche, die mit einem Fettsäureester behandelt wurde, um eine stark hydrophobe Oberfläche zu haben;
b. Platzieren von vom Patienten erhaltenen Zellen auf der hydrophoben Oberfläche des Substratmaterials, um autogene Zellen darauf zu kultivieren; und
c. Aufbringen von autogenem Plasma, das aus einer Blutprobe des Patienten erhalten wurde, auf die Zellen als Wachstumsmedium, um die Zellen zu kultivieren.

2. Verfahren nach Anspruch 1, wobei das Substratmaterial als Transferverband zum Übertragen der kultivierten Zellen auf den Patienten verwendet wird.

3. Verfahren nach Anspruch 1 oder Anspruch 2, wobei das Substratmaterial ferner als Wundabdeckverband verwendet wird.

4. Verfahren nach einem der vorhergehenden Ansprüche, wobei das Substratmaterial ein Celluloseacetat, eine Baumwollgaze oder ein Vliesgewebe aufweist und die Fettsäure ausgewählt ist aus einem Dialkyl-Carbamoyl-Chlorid (DACC) und einem Alkyl-Keten-Dimer (AKD).

5. Verfahren nach Anspruch 4, wobei das DACC Dihexadecyl-Carbamoyl-Chlorid oder Dioctadecyl-Carbamoyl-Chlorid ist.

6. Verfahren nach einem der vorhergehenden Ansprüche, wobei die Zellen aus Hautzellen oder Epithelzellen ausgewählt sind, die Fibroblasten und Melanozyten umfassen.

7. Verfahren nach einem der vorhergehenden Ansprüche, wobei das Plasma plättchenreiches Plasma (PRP) ist.

8. Verfahren nach einem der vorhergehenden Ansprüche, das einen weiteren Schritt des Befeuchtens der Zellen unter Verwendung eines Hydrogels mindestens alle 2 bis 4 Tage aufweist.

9. Kit zur Kultivierung autogener Zellen zur Verwendung bei der Behandlung eines Säugetierpatienten, wobei das Kit aufweist:
a. eine Trypsinlösung oder Lösungsvorläufer zur Trennung von epidermalen und dermalen Zellen einer von einem Patienten erhaltenen Biopsie
b. ein Substratmaterial mit einer Oberfläche, die mit einem Fettsäureester behandelt wurde, um eine stark hydrophobe Oberfläche zu haben, und auf der die Zellen platziert werden können, um autogene Zellen darauf zu kultivieren; und
c. eine Vielzahl von Röhren zur Aufnahme von Plasma, das aus einer Blutprobe des Patienten erhalten wurde.

10. Kit nach Anspruch 9, das weiterhin eine Mehrzahl Spritzen zum Extrahieren von Plasma aus einer vom Patienten erhaltenen Blutprobe aufweist; eine Mehrzahl von sterilen Nadeln und 15/0 Klingen (Theatermaterial) und eine Röhre mit Hydrogel.

11. Kit nach Anspruch 10 in Form eines unterteilten Behälters mit einer Mehrzahl von Zonen; umfassend eine erste Zone, die die Trypsinlösung oder Lösungsvorläufer enthält; eine zweite Zone, die das Substratmaterial und gegebenenfalls einen Stützrahmen zum Halten des Substratmaterials in einem abgeflachten Zustand enthält; eine dritte Zone, die die Röhren enthält; und eine vierte Zone, die die Spritzen, sterilen Nadeln und Klingen und das Hydrogel enthält; und wobei jede Zone einzeln durch eine abziehbare Filmabdeckung versiegelt ist.

12. Kit nach einem der Ansprüche 9 bis 11, das weiterhin eine Packungsbeilage enthält, die Anweisungen zur Verwendung des Kits zum Kultivieren autogener Zellen enthält.

## Revendications

1. Méthode de culture de cellules autogènes pour une utilisation dans le traitement d'un patient mammifère, les étapes comprenant :
a. fournir un matériau de substrat ayant une surface traitée par un ester d'acide gras de manière à avoir une forte surface hydrophobe ;
b. placer les cellules obtenues à partir du patient sur la surface hydrophobe du matériau de substrat, de manière à y cultiver des cellules autogènes ; et
c. appliquer du plasma autogène obtenu à partir d'un échantillon sanguin du patient aux cellules en tant que milieu de croissance afin de cultiver les cellules.

2. Méthode selon la revendication 1, dans laquelle le matériau de substrat est utilisé comme pansement de transfert pour transférer les cellules cultivées au patient.

3. Méthode selon l'une des revendications 1 ou 2, dans laquelle le matériau de substrat est en outre utilisé comme pansement de recouvrement de plaie.

4. Méthode selon l'une quelconque des revendications précédentes, dans laquelle le matériau de substrat comprend un acétate de cellulose, une gaze de coton ou un tissu non tissé, et l'acide gras est choisi parmi un chlorure de dialkyl carbamoyle (DACC) et un dimère d'alkylcétène (AKD).

5. Méthode selon la revendication 4, dans laquelle le DACC est le chlorure de dihexadécyl-carbamoyle ou le chlorure de dioctadécyl-carbamoyle.

6. Méthode selon l'une quelconque des revendications précédentes, dans laquelle les cellules sont choisies parmi des cellules cutanées ou des cellules épithéliales qui comprennent des fibroblastes et des mélanocytes.

7. Méthode selon l'une quelconque des revendications précédentes, dans laquelle le plasma est un plasma riche en plaquettes (PRP).

8. Méthode selon l'une quelconque des revendications précédentes, qui comprend une étape supplémentaire d'hydratation des cellules à l'aide d'un hydrogel, au moins tous les 2 à 4 jours.

9. Kit pour la culture de cellules autogènes pour une utilisation dans le traitement d'un patient mammifère, le kit comprenant :
a. une solution de trypsine ou des précurseurs de solution pour séparer les cellules épidermiques et dermiques d'une biopsie obtenue à partir d'un patient ;
b. un matériau de substrat ayant une surface traitée par un ester d'acide gras de manière à avoir une forte surface hydrophobe et sur laquelle les cellules peuvent être placées de manière à y cultiver des cellules autogènes ; et
c. une pluralité de tubes pour contenir du plasma obtenu à partir d'un échantillon sanguin du patient.

10. Kit selon la revendication 9, qui comprend en outre une pluralité de seringues pour extraire le plasma d'un échantillon sanguin obtenu à partir du patient ; une pluralité d'aiguilles stériles et de lames 15/0 (stock de théâtre) et un tube d'hydrogel.

11. Kit selon la revendication 10 sous la forme d'un contenant cloisonné avec une pluralité de zones ; comprenant une première zone contenant la solution de trypsine ou les précurseurs de solution ; une deuxième zone contenant le matériau de substrat et facultativement un cadre de support pour maintenir le matériau de substrat dans un état aplati ; une troisième zone contenant les tubes ; et une quatrième zone contenant les seringues, les aiguilles et lames stériles et l'hydrogel ; et dans lequel chaque zone est scellée individuellement par une pellicule détachable.

12. Kit selon l'une quelconque des revendications 9 à 11, qui comprend en outre une notice d'emballage contenant des instructions d'utilisation du kit pour la culture de cellules autogènes.
